# EUROPEAN PATENT APPLICATION

(11) **EP 1 428 525 A1**
(43) Date of publication of application: **16.06.2004**
(21) Application number: 03028009.3
(22) Date of filing: 05.12.2003
(51) Int. Cl.: A61K 9/14, A61K 47/12, A61K 9/08, A61K 31/60

(54) **Pharmaceutical preparations for the veterinary use containing acetylsalicylic acid in the form of buffered soluble powder**

(30) Priority: 11.12.2002 IT MI20022619
(71) Applicant: INDUSTRIA ITALIANA INTEGRATORI TREI S.P.A., I-41100 Modena (IT)
(72) Inventor: Grassigli, Giacomo, 41100 Modena (IT); Fezzi, Luigi, 41100 Modena (IT)
(74) Representative: Minoja, Fabrizio, Dr.

(57) **Abstract**

Veterinary compositions in the form of water-soluble powder containing acetylsalicylic acid as active ingredient in admixture with conventional carriers and excipients, characterized in that the contain salts able to induce alkaline hydrolysis and a system able to buffer the resulting solution.

## Description

The present invention relates to veterinary compositions in the form of water-soluble powder containing acetylsalicylic acid as active ingredient in admixture with conventional carriers and excipients, characterized in that they contain salts able to induce alkaline hydrolysis and a system able to buffer the resulting solution.

In recent years, the interest of zootechnics for ASA antinflammatory and antistress actions has remarkably increased.

Acetylsalicylic acid (ASA) has poor water solubility and markedly acidic character. The low solubility of acetylsalicylic acid prevents it from being used in preparations designed to be delivered through drinking water. Moreover, its high acidity in solution affects the chemical properties of the water, generating conditions which prevent other substances from being delivered at the same time, and increases the risk of side effects affecting the gastric mucosa.

The medicinal products which have been launched on the market to date have solved the problem of the solubility of acetylsalicylic acid, albeit with considerable differences between them (water-dispersible and soluble formulations), making it possible to use them in drinking water, but these preparations have proved unsuitable to correct the acidity of the solution obtained.

The solutions proposed by the veterinary pharmaceutical industry to date do not allow the use of ASA together with vitamin supplements; treatments with beta-lactam antibiotics, sulphamides, or other substances in general which require a neutral or basic environment to dissolve in water.

Moreover, administration to calves in reconstituted powdered milk involves the risk of curdling.

The therapeutic limitation induced by the risk of ulcers caused by the acidity of the solution thus represents a limiting factor on the use of ASA-based medicaments in veterinary medicine.

The attention paid by the human drug industry to formulations buffered to limit and/or exclude the gastric complications associated with the use of ASA demonstrates the importance of controlling the acidity expressed by ASA.

It has thus been prepared a soluble powder formulation of acetylsalicylic acid with the following characteristics:
- Rapid, total solubilisation in the final carrier (drinking water or powdered milk).
- Efficient buffering effect against the acidity generated by the solubilisation of ASA.
- Stability of the formulation and stability of the active ingredient after solubilisation in water.

The need to deal jointly with the issues described above is partly due to the fact that the use of alkalinising salts in the formulation can lead to problems with the stability of the formulations.

A system of excipients which makes the preparation totally soluble in water and gives it an efficient buffering power and sufficient stability has now been found.

Therefore the present invention relates to water-soluble veterinary compositions containing acetylsalicylic acid as active ingredient, in admixture with conventional carriers and excipients, characterized in that they further contain a solubilising system and a buffer mixture, able to guarantee the complete solubilisation of the active ingredient in water as well as a buffering effect in the resulting solution.

According to the present invention, the excipient system should guarantee:
1) Dissolution of ASA due to the alkalinising effect induced by alkaline hydrolysis salts.
2) Fast wettability of the powder by means of a surfactant.
3) Buffering effect induced by mixtures of salts able to buffer drinking water to pH above 4.75.
4) Stability of the formulation and of the active ingredient in drinking water.

In an embodiment of the present invention, the solubilising system consists of one or more salts able to induce alkaline hydrolysis. The salts are preferably selected from the group consisting of anhydrous or hydrate sodium citrate and/or anhydrous or hydrate potassium citrate and/or sodium borate.

Powder wettability is promoted when using high HLB surfactants, such as sodium lauryl sulfate and sodium dioctyl sulfosuccinate, in the powdered or liquid form: in the latter case, the surfactant is applied to the powder by a conventional spraying procedure.

The buffer system consists of a salts couple in suitable ratio to induce a buffering effect. The buffer system preferably consists of one of the following couples: sodium citrate/citric acid, potassium citrate/citric acid, sodium monoacid phosphate/sodium diacid phosphate. A particularly preferred buffering composition is the sodium citrate/citric acid mixture in a 1:1 to 10:1, more preferably 3:1, ratio.

The formulations of the invention can further contain technological excipients such as alkaline-earth metals chelating agents, silica and derivatives and can be subjected to anti-dusting or granulation treatments using the suitable anti-dusting agents or ligands. Said excipients will be selected from those conventionally used in the veterinary medicine (Lactose, Dextrose, Sodium carbonate and the like).

The following examples further illustrate the invention.

| EXAMPLE 1 | |
|---|---|
| Acetylsalicylic acid | 66.0 |
| Citric acid | 6.0 |
| Sodium Citrate | 5.0 |
| Silica | 3.5 |
| Sodium lauryl sulfate | 1.0 |
| Sodium Carbonate q.s. to | 100 |

| EXAMPLE 2 | |
|---|---|
| Acetylsalicylic acid | 66.0 |
| Citric acid | 4.0 |
| Sodium Citrate | 11.5 |
| Silica | 3.5 |
| Sodium dioctyl sulfosuccinate | 1.0 |
| Sodium Carbonate q.s. to | 100 |

| EXAMPLE 3 | |
|---|---|
| Acetylsalicylic acid | 70.0 |
| Citric acid | 4.0 |
| Silica | 3.5 |
| Sodium lauryl sulfate | 1.0 |
| Potassium citrate | 12.5 |
| Sodium monoacid phosphate | 1.5 |
| Sodium phosphate diacid | 5.5 |
| Sodium edetate | 1.0 |
| Dextrose q.s. to | 100 |

| EXAMPLE 4 | |
|---|---|
| Acetylsalicylic acid | 50.0 |
| Citric acid | 5.0 |
| Silica | 3.5 |
| Sodium lauryl sulfate | 5.0 |
| Sodium citrate | 5.0 |
| Sodium Borate | 6.0 |
| Sodium monoacid phosphate | 2.5 |
| Sodium phosphate diacid | 7.0 |
| Sodium edetate | 1.0 |
| Polyvinyl pyrrolidone | 5.0 |
| Lactose q.s. to | 100 |

| EXAMPLE 5 | |
|---|---|
| Acetylsalicylic acid | 50.0 |
| Citric acid | 5.0 |
| Silica | 3.5 |
| Sodium lauryl sulfate | 1.0 |
| Sodium Citrate | 10.0 |
| Sodium Borate | 6.0 |
| Sodium monoacid phosphate | 2.5 |
| Sodium phosphate diacid | 7.0 |
| Sodium edetate | 1.0 |
| Polyvinyl pyrrolidone | 5.0 |
| Dextrose q.s. to | 100.0 |

## Claims

1. Veterinary compositions in the form of water-soluble powder containing acetylsalicylic acid as active ingredient in admixture with conventional carriers and excipients, **characterized in that** they contain salts able to induce alkaline hydrolysis and a system able to buffer the resulting solution.

2. Compositions as claimed in claim 1, wherein the buffering system consists of salts mixtures able to buffer the pH of the solution to pH above 4.75.

3. Compositions as claimed in claim 2, wherein the salts able to induce alkaline hydrolysis are selected from the group consisting of anhydrous or hydrate sodium citrate, anhydrous or hydrate potassium citrate, anhydrous or hydrate sodium borate.

4. Compositions as claimed in claim 2 or 3 wherein the buffering system is selected from sodium citrate/citric acid, potassium citrate/citric acid, sodium monoacid phosphate/sodium diacid phosphate, potassium monoacid phosphate/potassium diacid phosphate.

5. Compositions as claimed in claim 4 wherein said buffering system consists of sodium citrate/citric acid.

6. Compositions as claimed in claim 5 wherein the sodium citrate to citric acid ratio ranges from 1:1 to 10:1.

7. Compositions as claimed in the above claims further containing wetting surfactants.

8. Compositions as claimed in claim 7 wherein the su rfactant is selected from sodium lauryl sulfate and sodium dioctyl sulfosuccinate.

9. Compositions as claimed in the above claims subjected to anti-dusting and/or granulation treatments.

10. A composition as claimed in the above claims having the following composition:
| | |
|---|---|
| Acetylsalicylic acid | 66.0 |
| Citric acid | 6.0 |
| Sodium Citrate | 5.0 |
| Silica | 3.5 |
| Sodium lauryl sulfate | 1.0 |
| Sodium Carbonate q.s. to | 100 |

11. A composition as claimed in claims 1-9, having the following composition:
| | |
|---|---|
| Acetylsalicylic acid | 50.0 |
| Citric acid | 5.0 |
| Silica | 3.5 |
| Sodium lauryl sulfate | 1.0 |
| Sodium Citrate | 10.0 |
| Sodium Borate | 6.0 |
| Sodium monoacid phosphate | 2.5 |
| Sodium phosphate diacid | 7.0 |
| Sodium edetate | 1.0 |
| Polyvinyl pyrrolidone | 5.0 |
| Dextrose q.s. to | 100.0 |

12. Compositions as claimed in the above claims containing lactose, dextrose and sodium carbonate as excipients.
